# EUROPEAN PATENT SPECIFICATION

(11) **EP 1 685 869 B1**
(45) Date of publication and mention of the grant of the patent: **27.02.2008**
(21) Application number: 06250319.8
(22) Date of filing: 20.01.2006
(51) Int. Cl.: A61M 25/09

(54) **A medical guide wire**
Medizinischer Führungsdraht
Fil de guidage médical

(30) Priority: 26.01.2005 JP 2005017573
(43) Date of publication of application: 02.08.2006
(73) Proprietor: ASAHI INTECC CO., LTD., Nagoya-shi, Aichi-ken (JP)
(72) Inventor: Kato, Tomihisa, Aichi-ken (JP)
(74) Representative: Senior, Alan Murray

(56) References cited:
- EP-A- 1 576 980
- EP-B- 0 803 266
- US-A- 5 253 653
- US-A- 5 497 783
- US-A1- 2004 087 876

## Description

The invention relates to a medical guide wire used to assist a catheter upon inserting it into a somatic cavity to examine and treat a diseased area (e.g., vascular stenosis) and measuring a dimensional size of the diseased area.

In a medical guide wire (referred sometimes to as " guide wire" hereinafter) which is provided to introduce a leading distal end into a diseased area through a sinuous vascular system, the leading distal end of the guide wire is inserted into the blood vessel or the somatic cavity to implement a "push-pull and turn" manipulation at a proximal portion located ouside a subject patient upon treating his or her diseased area.

In order to achieve a smooth manipulation upon inserting the leading distal end into the somatic cavity and the blood vessel, it is required for the guide wire to have multi-mechanical properties. The multi-mechanical properties include a high flexibility, a good straightness secured in unrestricted, free state and a good restitutivity from the manipulative deformation. The guide wire of this type is required at its leading distal end portion to have a high flexibility, while at the same time, being required at its rear portion to have an appropriate rigidity as a functionally gradient property. It is also indispensable for the leading distal end to have a high steerability in which the leading distal end properly responds to the manual operation which is to be done outside the subject patient.

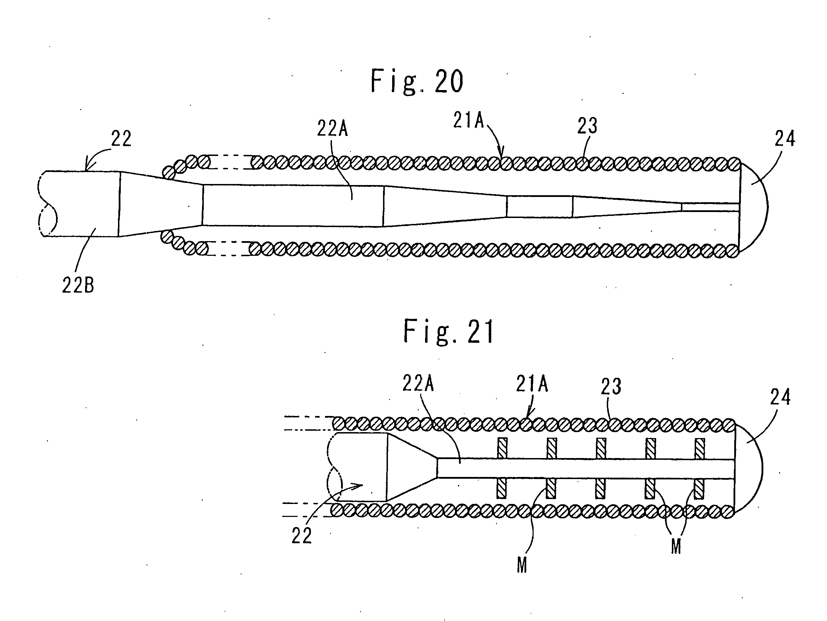

As shown in Fig. 20, a medical guide wire generally has a flexible elongation core 22 at its leading end portion 21A which has a distal front portion 22A and a proximal portion 22B provided to be diametrically larger than the distal front portion 22A. The distal front portion 22A has a helical spring tube 23, both ends of which are secured to the flexible elongation core 22 as a basic structure of the medical guide wire.

Among guide wires of the basic structure, U.S. Patent No. 5,497,783 discloses a guide wire in which a helical spring tube and an elongation core are integrally connected at regular intervals by means of soldering in the axial direction.

Japanese Laid-open Patent Application No. 8-173547 discloses a guide wire in which only one fixedly-connected portion (small in breadth) is provided between an inner surface of a helical spring tube and an outer surface of an elongation core.

EP0803266 discloses a guide wire in which also only one fixedly-connected portion is provided.

Japanese Domestic Publication No. 7-500749 discloses a guide wire similar to another counterpart shown in Fig. 21. The guide wire provides markers M at spaced intervals along the distal front portion 22A of the elongation core 22. The markers M are secured to the inner surface of the helical spring tube 23 to provide a clearance with the outer surface of the elongation core 22. Alternatively, the markers M are secured to the outer surface of the elongation core 22 to provide a clearance with the inner surface of the helical spring tube 23. The guide wire has a size measuring function in which the radioactive projection enables the manipulator to dimensionally measure sizes of the diseased area with the markers M.

Japanese Domestic Publication No. 2004-516049 discloses a guide wire in which a distal front portion of an elongation core has radiopaque markers at spaced intervals.

U.S. Patent No. 5,797,856 discloses a guide wire in which a distal front portion of an elongation core, a helical spring tube and a tubular portion are secured by means of soldering.

These related art guide wires only concerns to the structural arrangement of markers within the helical spring tube and the securement between the helical spring tube and the elongation core based on their individual purposes. This deteriorates a bending characteristics upon navigating the leading end portion 21A along a complicatedly curved path within the somatic cavity. This holds true upon selectively inserting the leading end portion 21A into bifurcated portions of the blood vessel. This also deteriorates the steerability of the leading end portion 21A upon manipulating the "push-pull and turn" operation at the proximal portion located ouside a subject patient upon treating the diseased area. It is by no means easy for the manipulator to achieve good results upon making use of the size measuring function of the markers.

Especially the characteristics reduces at the steerability and insertability upon deeply navigating the leading end portion 21A (bent generally at right angle) from the left main trunk (LMT) to the left anterior descending artery (LAD), while at the same time, the measuring capability deteriorates upon dimensionally measuring the diseased area residing at the left anterior descending artery (LAD). It is to be noted that reasons why the bending and maneuverable characteristics deteriorate are supplementarily mentioned in detail hereinafter.

Therefore, it is an object of the invention to overcome the above drawbacks so as to provide a medical guide wire of a high quality and high performance which is capable of treating the diseased area significantly well with a high rotation-following capability, excellent torque transmissiblity and enhanced steerability.

### SUMMARY OF THE INVENTION

According to the present invention, there is provided a medical guide wire including a flexible elongation core having a distal front portion, a proximal portion provided to be diametrically larger than the distal front portion and a leading front portion around which a helical spring tube is provided, both ends of which are secured to the flexible elongation core. The distal front portion of the flexible elongation core is diametrically tapered or reduced progressively as approaching toward a distal end of the flexible elgonation core. A non-integral region is provided to form an annular space between the flexible elongation core and the helical spring tube to extend at least 20 mm axially from the distal end of the flexible elongation core. An intermediate region is provided to form a group of fixedly-connected portions between the flexible elongation core and the helical spring tube to axially extend from 50 to 125 mm from the distal end of the flexible elongation core. A proximal region is provided to form a group of fixedly-connected portions between the flexible elongation core and the helical spring tube to axially extend from 125 to 300 mm from the distal end of the flexible elongation core. Spans between the fixedly-connected portions of the proximal region are greater than spans between the fixedly-connected portions of the intermediate region. The fixedly-connected portions are formed into a doughnut-shaped configuration to have 0.3 to 1.5 mm in breadth, and integrally connecting an inner surface of the helical spring tube to an outer surface of the flexible elongation core, and other limitations are set out in claim 1.

With the elongation core and the helical spring tube concentrically secured integrally through the fixedly-connected portions, it is possible to unite the helical spring tube and the elongation core integrally to form a flexible elongated one piece structure so as to make it mechanically stable against the bending force and the rotational force. This makes it possible to impart a high steerability and good bending characteristics to the leading front portion of the medical guide wire.

According to other aspect of the present invention, the spans between the fixedly-connected portions of the intermediate region are arranged to be progressively reduced or increased dimensionally in a series fashion (arithmetical series or geometrical series) along an axial direction of the flexible elongation core.

According to other aspect of the present invention, the fixedly-connected portions of the intermediate region are formed by a radiopaque material and arranged at regular intervals, and a front half of the helical spring tube and a rear half of the helical spring tube are made by different metals of a radiopaque material and a radiotransparent material, the different metals being connectedly bonded and wound to form a single helical structure. The front half of the helical spring tube is of the radiopaque material and having a helical length integral times greater than the span of the intermediate region.

According to other aspect of the present invention, the fixedly-connected portions of the intermediate region are formed by a radiopaque material to provide a plurality of unit portions composed of smaller spans and larger spans, and a front half of the helical spring tube and a rear half of the helical spring tube are made by different metals of a radiopaque material and a radiotransparent material so as to form a single helical structure. The front half of the helical spring tube is of the radiopaque material and having a helical length integral times greater than the smaller spans.

According to other aspect of the present invention, spans of the fixedly-connected portions of the intermediate region forms a plurality of the unit portions composed of larger spans at proximal side of the flexible elongation core and smaller spans at distal side of the flexible elongation core.

According to other aspect of the present invention, the number of the fixedly-connected portions of the proximal region is in the range of 1-3.

According to other aspect of the present invention, a space opposed interval of an opposed pair of the fixedly-connected portions axially arranged along the flexible elongation core is determined with a diametrical dimension as a reference level in which the opposed pair of the fixedly-connected portions are located at the flexible elongation core, and forming a structure which retains a uniform torque transmissibility and the rotation-following capability, or forming a structure which gradually decreases the torque transmissibility and the rotation-following capability from a proximal side to a distal side of the flexible elongation core.

### BRIEF DESCRIPTION OF THE DRAWINGS

Fig. 1 is a longitudinal cross sectional view of a medical guide wire according to a first embodiment of the invention;
Fig. 2 is a latitudinal cross sectional view of the medical guide wire taken along the line II-II of Fig. 1;
Fig. 3 is an explanatory view showing how a helical spring tube is manipulated;
Fig. 4 is an explanatory view showing how a prior art helical spring tube is manipulated;
Fig. 5 is a longitudinal cross sectional view of a main portion of a medical guide wire according to a second embodiment of the invention;
Fig. 6 is a longitudinal cross sectional view of a main portion of the medical guide wire;
Fig. 7 is a longitudinal cross sectional view of a main portion of a medical guide wire according to a third embodiment of the invention;
Fig. 8 is an explanatory view of the medical guide wire in use;
Fig. 9 is a longitudinal cross sectional view of a main portion of a medical guide wire according to a fourth embodiment of the invention;
Figs. 10 and 11 are explanatory views of the medical guide wire in use;
Fig. 12 is a longitudinal cross sectional view of a main portion of a medical guide wire according to a fifth embodiment of the invention;
Fig. 13 is an explanatory view of the medical guide wire;
Fig. 14 is a graphical representation of a rotational torque characteristics between a rotational angle of a proximal end portion and a rotational angle of a distal end portion;
Fig. 15 is an explanatory view of the medical guide wire in use;
Figs. 16 through 19 are schematic views of opposed intervals of a pair of fixedly-connected portions shown to determine dimensional relationships; and
Figs. 20 and 21 are longitudinal cross sectional views of main portions of related art medical guide wires.

### DETAILED DESCRIPTION OF THE PREFERRED EMBODIMENTS

In the following description of the depicted embodiments, the like reference numerals are used for features of the same type.

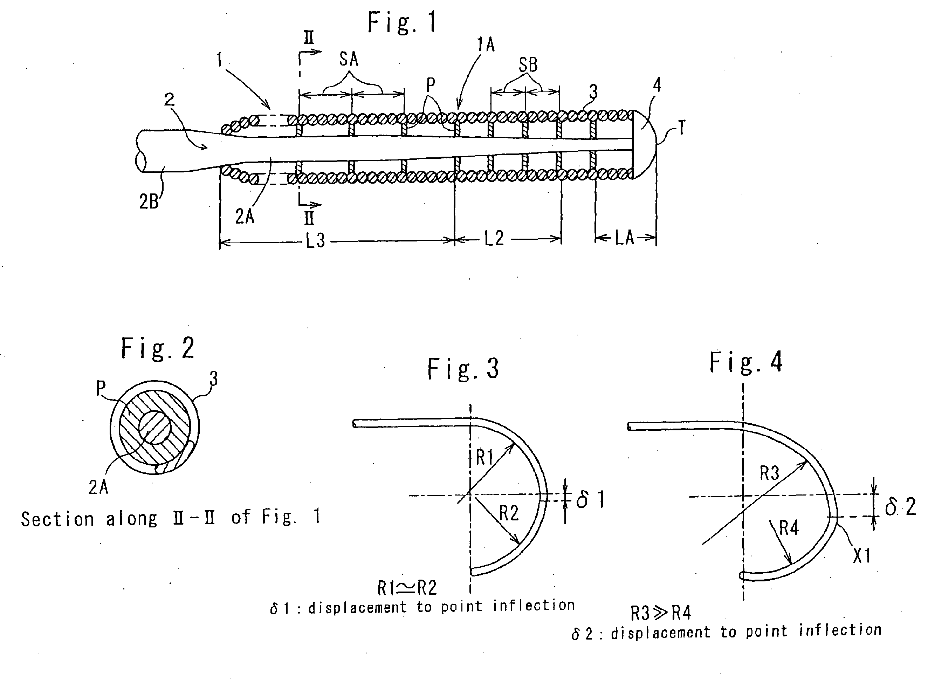

Referring to Figs. 1 through 4, a medical guide wire 1 is provided according to a first embodiment of the invention. The medical guide wire 1 includes a flexible elongation core 2 having a distal front portion 2A, a proximal portion 2B provided to be diametrically larger than the distal front portion 2A and a leading front portion 1A, around which a helical spring tube 3 is provided, both ends of which are secured to the flexible elongation core 2.

The distal front portion 2A of the flexible elongation core 2 is diametrically tapered or reduced progressively as approaching toward a distal end T of the flexible elongation core 2. A non-integral region (LA) is provided to form an annular space between the flexible elongation core 2 and the helical spring tube 3 to axially extend by at least 20 mm from the distal end T of the flexible elongation core 2. An intermediate region (L2) is provided to form a group of fixedly-connected portions P between the flexible elongation core 2 and the helical spring tube 3 to axially extend from 50 to 125 mm from the distal end T of the flexible elongation core 2. Region L2 is not continuous with region LA in this case, but it could be.

A proximal region (L3) is provided to form a group of fixedly-connected portions P between the flexible elongation core 2 and the helical spring tube 3 to axially extend from 125 to 300 mm from the distal end T of the flexible elongation core 2. Spans between the fixedly-connected portions P of the proximal region (L3) are greater than spans appeared between the fixedly-connected portions P of the intermediate region (L2).

The fixedly-connected portions P are formed into a doughnut-shaped configuration to have 0.3- 1.5 mm in breadth, and integrally connecting an inner surface of the helical spring tube 3 to an outer surface of the flexible elongation core 2.

With the flexible elongation core 2 and the helical spring tube 3 concentrically secured integrally through the fixedly-connected portions P, it is possible to unite the helical spring tube 3 and the flexible elongation core 2 integrally to form a flexible elongated one piece structure so as to make it mechanically stable against the bending force and the rotational force. This makes it possible to impart a high steerability and good bending characteristics to the leading front portion 1A of the medical guide wire 1.

The reason why the non-integral region (LA) axially extends by at least 20 mm from the distal end T of the flexible elongation core 2 is to easily preshape a leading end portion of the guide wire 1 into a dog-legged configuration with fingertips upon inserting the guide wire 1 into the somatic cavity, while at the same time, providing a high flexibility with the guide wire 1 to insure a smooth insertion against the somatic cavity. The non-integral region (LA) defines an annular space area free from the fixedly-connected portions P between the flexible elongation core 2 and the helical spring tube 3.

It is to be noted that the distal front portion 2A of the flexible elongation core 2 and the helical spring tube 3 are preferably secured through the fixedly-connected portions P in a concentric relationship each other. However, the concentricity between the distal front portion 2A and the helical spring tube 3 needs not always precise since the fixedly-connected portions P are formed within the miniature helical spring tube 3 upon putting into a mass production.

Based on the medical guide wire 1 of the invention, the following advantages are obtained.

By comparing the guide wire 1 to a prior art guide wire 20 in which the fixedly-connected portions P is not provided as shown in Figs. 3 and 4 when they are bent into a U-shaped configuration with a common radius of curvature, it is found that the guide wire 20 deforms while gradually decreasing the radius of curvature from the proximal side to the distal side as designated at R3, R4. The deformation depends on the bending rigidity based on the tapered configuration of the distal front portion of the flexible elongation core. Upon changing the radius of curvature from R3 to R4, a boundary section between R3 and R4 continuously projects outward as a point of inflection X1 to form an irregular U-shaped configuration.

As contrast to the prior art guide wire 20, the guide wire 1 smoothly shifts the radius of curvature from R1 to R2 without inviting the point of inflection as shown in Fig. 3, when the guide wire 1 is bent while gradually decreasing the radius of curvature from the proximal side to the distal side as designated at R1, R2.

Under the presence of the fixedly-connected portions P, it is possible for the guide wire 1 to stabilize a relative position between the flexible elongation core 2 and the helical spring tube 3 against a neutral plane (central line of the flexible elongation core 2. This stabilizes a moment of inertia of the flexible elongation core 2 along the leading front portion 1A of the guide wire 1.

In the meanwhile, the prior art guide wire 20 shifts the neutral plane away from the center of the flexible elongation core due to a bending resistance, to which the guide wire 20 is subjected when bent. This permits a relative movement between the helical spring tube and the flexible elongation core so as to reduce the moment of inertia when the flexible elongation core is bent only to invite the point of inflection X1.

Since the guide wire 20 has the point of inflection X1 which projects outward, it causes to forcibly expand the vascular wall to increase the resistance when inserted into the blood vessel only to injure the vascular wall and increase the burden which the subject patient owes.

As opposed to the prior art guide wire 20, the bending operation enables the manipulator to smoothly deform the guide wire 1 free from the point of inflection to overcome the above drawbacks so as to significantly ameliorate the treatment against the diseased area.

Further, the guide wire 1 has a steerability significantly improved when inserted into the somatic cavity. As a general rule, the torsional angle is in direct proportion to turns of the helical spring and the rotational torque is in inverse proportion to the turns of the helical spring when both ends of the helical spring is subjected to a torsional force.

Since the helical spring tube 3 is lengthwisely divided into a plurality of compartments by the fixedly-connected portions P, each of the compartments is subjected to a uniform rotational torque so that each compartment deforms based on the above general rule so as to achieve a high rotation-following capability.

Similar to the rotational torque, each of the compartments is subjected to a uniform transmission of the rotation torque. This provides the guide wire 1 with a high torque transmissibility. The torque transmitted from the proximal side to the distal side in the guide wire 1 is 3-5 times as great as the torque in the guide wire 20 in which the fixedly-connected portions P are not provided.

With the high rotation-following capability and the high torque transmissibility insured for the guide wire 1, it is possible to enhance the steerabilty so as to significantly ameliorate the treatment against the diseased area.

The technological concept under the presence of the fixedly-connected portions P is apparently different from that of the related art in which the flexible elongation core and the helical spring tube are diametrically thicken only to increase their rigidity.

With the fixedly-connected portions P adjustable at any intervals, it is possible to increase the bending rigidity of the leading front portion 1A by reducing the span between the fixedly-connected portions P. Conversely, it is possible to decrease the bending rigidity of the leading front portion 1A by increasing the span between the fixedly-connected portions P. For this reason, the fixedly-connected portions P makes the bending rigidity ajustable to the bendable limit curvature of the guide wire 1 when bent due to the normal pushing manipulation.

At the bifurcated portions and tortuously curved path of the blood vessel in which the guide wire is likely to deform abnormally, the guide wire 1 makes it possible for the proximal side to detect an abnormal resistance from the above portions. This enables the manipulator to detect the abnormal pushing and rotational manipulation and retains the pushing and rotational manipulation within a reasonable bound, thus obviating an injury on the vascular wall, a rupture on the vascular wall and a damage on the guide wire 1 so as to insure a smooth navigation into the blood vessel due to the normal manipulation force.

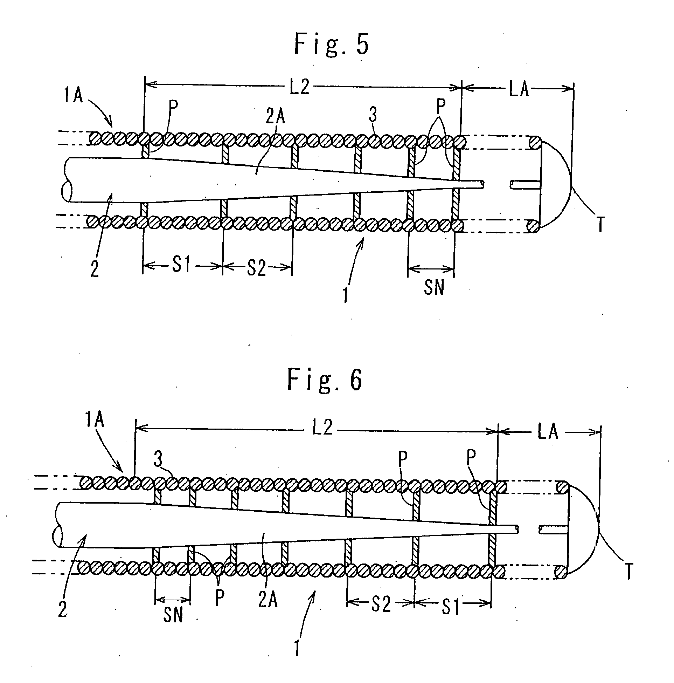

Figs. 5 and 6 show a second embodiment of the invention in which a group of the fixedly-connected portions P are provided in the intermediate region (L2).

In the guide wire 1 of Fig. 5, spans S1, S2, S3, ··· , SN appeared between the fixedly-connected portions P progressively decrease from a rear end side to a front end side along the distal front portion 2A. In this instance, the spans form an arithmetical series as exemplified by S1 = 25mm, S2 = 20mm, S3 = 15mm, ··· , SN = 25-5(N-1) mm.

In the guide wire 1 of Fig. 6, spans S1, S2, S3, ... , SN appeared between the fixedly-connected portions P progressively increase from a rear end side to a front end side along the distal front portion 2A. By way of illustration, the spans form a geometrical series as exemplified by S1 = 40mm, S2 = 20mm, S3 = 10mm, ···, SN = 40(1/2) ^{N-1} mm.

In this situation, an entire length of the guide wire 1 is 1500 mm, a length of the leading front portion 1A is approx. 300 mm. The distal front portion 2A of the flexible elongation core 2 is 0.193 mm in diameter at the proximal side, and 0.03 mm in diameter at the distal side. The helical spring tube 3 is 0.355 mm in outer diameter and having a helical diameter determined as 0.072 mm. The flexible elongation core 2 and the helical spring tube 3 are formed by a stainless steel wire. In the intermediate region (L2) continuous from the non-integral region (LA), the fixedly-connected portions P which are formed into the doughnut-shaped configuration are secured connectedly between the outer surface of the elongated core 2 and the inner surface of the helical spring tube 3 by means of brazing procedure (e. g., gold-based alloy).

By selectively determining the spans S1 S2, S3, ... , SN (arithmetrical series or geometrical series), it is possible to delicately shift the bending characteristics derived from the leading front portion 1A of the guide wire 1, thus matching the maneuverability to the diseased area and the individual skills of the manipulators to produce a wide variety of guide wires so as to significantly ameliorate the treatment against the diseased area.

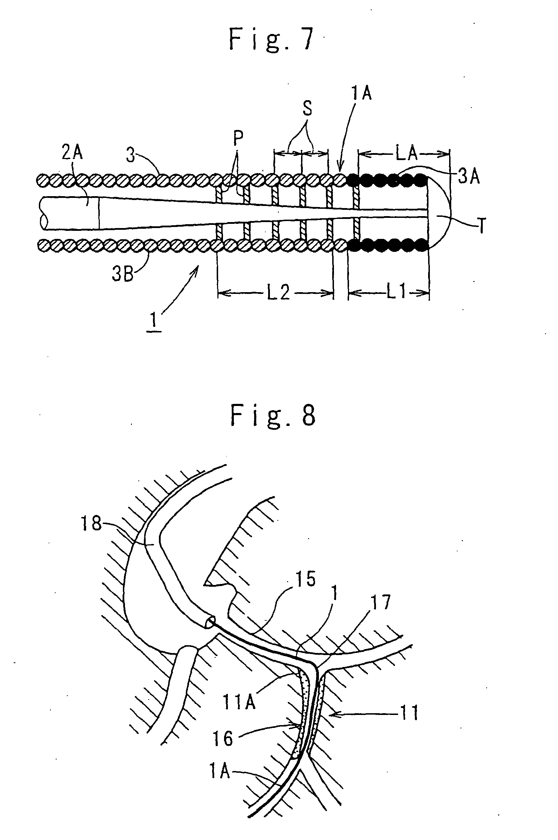

Figs. 7 and 8 show a third embodiment of the invention in which the guide wire 1 has a size measuring function.

In this instance, the helical spring tube 3 has a radiopaque front portion 3A which has 30 mm in length (L1). The fixedly-connected portions P are formed by a radiopaque material and arranged at regular intervals as small spans (S) in the intermediate region (L2). The length (L1) of the front portion 3A is integral times as great as the spans (S). The front portion 3A and the spans (S) work as a large, medium and small graduation rulers for a size measuring device. Under the presence of the fixedly-connected portions P, the intermediate region (L2) especially enhances the rotation-following capability for the flexible elongation core 2 as described in detail hereinafter.

The front portion 3A of the helical spring tube 3 and a rear portion 3B of the helical spring tube 3 are made by different metals of a radiopaque material and a radiotransparent material so as to form a single one helical structure. Upon making the helical spring tube 3, a platinum wire and a stainless steel wire are firmly connected in tandem by means of welding, and are drawn until they are thinned to be 0.072 mm in diameter.

Due to the length (L1) of the front portion 3A and the fixedly-connected portions P, it is possible to measure dimensional sizes of the diseased area and disease-related portions on the radioactive projection plane upon injecting the contrast medium into the somatic cavity.

Since the length (L1) of the front portion 3A is integral times as great as the small spans (S), it is possible to highly precisely measure dimensional sizes of the blood vessel by comparing the front portion 3A with the small spans (S) appeared between the fixedly-connected portions P on the radioactive projection plane, however complicatedly and tortuously the blood vessel is curved in three dimensions. It is to be noted that the fixedly-connected portions P may be formed into the doughnut-shaped configuration by melting a radiopaque metal ball (gold brazing, silver brazing, tungsten brazing or the like). The fixedly-connected portions P are concentrically secured integrally to the outer surface of the flexible elongation core 2 and the inner surface of the helical spring tube 3.

Since the helical spring tube 3 is made of different metallic materials, an amount of the springback differs between the front portion 3A and the rear portion 3B upon winding the platinum wire and the stainless steel wire to form them into helical spring tube 3.

Due to the springback difference between the two portions 3A, 3B, it is possible to influence the front portion 3A to diametrically reduce so that the leading front portion 1A decreases its outer diameter progressively as approaching toward the distal end T of the flexible elongation core 2 so as to substantially form a tapered-off structure. This contributes to helping the leading front portion 1A penetrate into the vascular stenosis portion, the intima and the media so as to ameliorate the treatment of the diseased area.

Since the guide wire 1 has the size measuring function and the tapered-off structure, it is especially advantageous in treating the coronary artery. Namely, in the coronary artery, the most of the diseased areas are found at the bifurcated portions of the blood vessel, and the guide wire 1 is inserted into the coronary artery by 100-125 mm from the distal end T with the use of a catheter 18. In the left main trunk (LMT) 15, the diseased area (e.g., vascular stenosis 11) is likely to appear when inserted by 30-60 mm from an entrance as shown at numeral 16 in Fig. 8.

Upon measuring the vascular stenosis 11, it is only 30-60 mm from the entrance of the left anterior descending artery (LAD) 15 that is an insertable length for the guide wire disclosed by the Japanese Domestic Publication No. 7-500749 which measures the diseased area within approx. 50 mm from the distal end of the leading front portion. This unsteadily fluctuates the leading front portion of the guide wire when exposed to the rapid blood streams, thus impeding the guide wire from precisely measuring the diseased area.

As opposed to the above structure, it is possible to navigate the guide wire 1 beyond the vascular stenosis 11 deep into the artery since the helical spring tube 3 is progressively reduces its diameter as approaching toward the distal end T of the leading front portion 1A. With the fixedly-connected portions P extending by 300 mm from the distal end T of the leading front portion 1A, it is possible to stabilize the leading front portion 1A even when inserted deep into the artery so as to dimensionally measure the diseased area with a high precision. This is done especially by placing the fixedly-connected portions P {notation 17 in the intermediate region (L2) } at one end 11A of the vascular stenosis 11 so as to avoid the leading front portion 1A from being fluctuated due to the rapid blood streams.

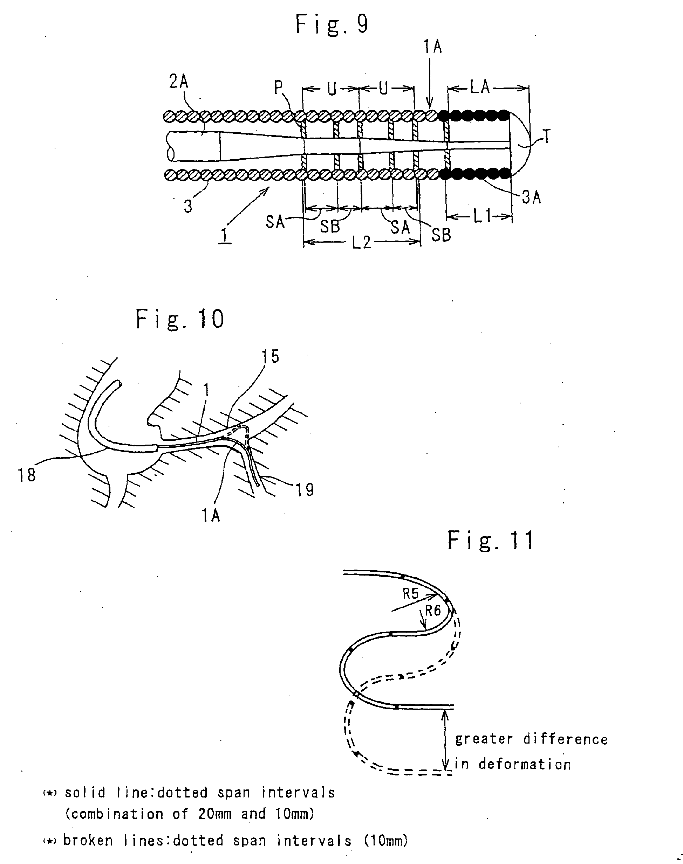

Figs. 9 through 11 show a fourth embodiment of the invention which differs from the third embodiment in that the a plurality of unit portions U are provided in the intermediate region (L2). Each of the unit portions U is a combination of a larger span (SA) in the proximal side and a smaller span (SB) in the distal side. The radiopaque front portion 3A has the length (L1) integral times as great as the smaller span (SB). It is to be noted that although the larger span (SA) preferably resides in the proximal side because the elongated core 3 becomes thicker and higher in rigidity as approaching the proximal side, one of the larger spans (SA) may reside in the distal side.

Upon manipulating the leading front portion 1A of the guide wire 1 to advance it into the left anterior descending artery (LAD) 19 from the left main trunk (LMT) 15, the manipulation abruptly changes the leading front portion 1A generally at right angle as shown in Fig. 10.

When the spans (S) terminate short of 10 mm, the spans (S) work the leading front portion 1A to maintain a certain radius of curvature as shown at the broken lines in Fig. 10, thus making it difficult to further deform the leading front portion 1A in the bending direction. In this situation, when the leading front portion 1A is forcibly pushed, the leading front portion 1A is stuck in the artery or would do damage on the vascular wall due to a reactionary force appeared when forcibly pushed.

On the contray, the guide wire 1 can determine the smaller span (SB) to be 10 mm in length and the larger span (SA) to be 20 mm in length. This makes it possible to smoothly advance the leading front portion 1A into the left anterior hemlock 19, thus ameliorating the insertability against the bifurcated portions curved substantially at right angle so as to insure an excellent rotational and pushing manipulations concurrently.

As opposed to the guide wire 1 in which the fixedly-connected portions P are arranged at regular spans (S), and the leading front portion 1A has a tendency to maintain a certain radius of curvature as shown at the broken lines in Fig. 11, the guide wire 1 forms a small radius of curvature R5 on the leading front portion 1A due to the larger span (SA) and a large radius of curvature R6 due to the smaller span (SB) when bent under the presence of the unit portion U as shown at the solid line in Fig. 11.

This enables the manipulator to smoothly insert the leading front portion 1A into an extremely curved artery, while at the same time, preventing the leading front portion 1A from abruptly bent abnormally.

For this reason, the guide wire 1 based on the fourth embodiment of the invention becomes suited to treating the left anterior hemlock 19 which develops a diffuse lesion area (longer than 20 mm) and invites an abnormal resistance felt when inserting the guide wire 1 into the left anterior descending artery (LAD) 19. With the fixedly-connected portions P in the unit portions U formed by the radiopaque material, it is possible to dimensionally measure a longer disease portion (e.g., diffuse lesion area) with a high precision.

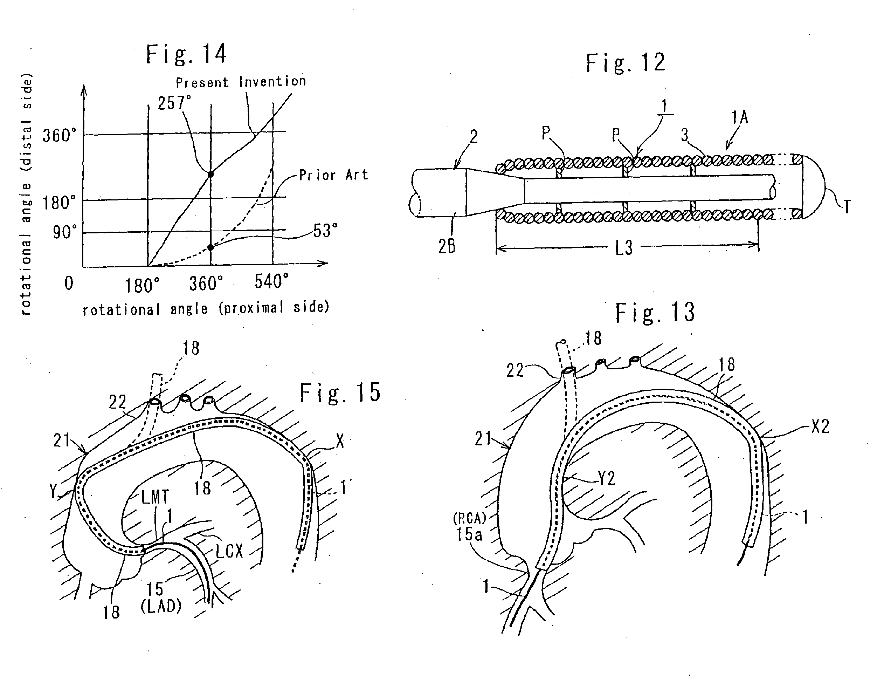

Figs. 12 through 15 show a fifth embodiment of the invention in which number of the fixedly-connected portions P is 1-3. The fixedly-connected portions P are provided within the proximal region (L3) situated by 125-300 mm rearward from the distal end T of the elongated core 2.

The fixedly-connected portions P thus structured enables the manipulator to attain a good rotation-following capability felt when inserting the guide wire 1 into the coronary artery.

Upon inserting the guide wire 1 and the catheter 18 into the aortic arch 21 of the left main trunk (LMT) 15, the catheter 18 leads the distal end portion to an entrance of the left main trunk (LMT) 15. The manipulation advances the guide wire 1 through the catheter 18 with an reactionary force carried by the catheter 18 while rotating the guide wire 1 fifteen times within the left main trunk (LM T) 15.

In this situation, the catheter 18 curvedly deforms to make their elevational portions in contact with the vascular wall at points X and Y, thus increasing the rotational resistance of the guide wire 1 to produce different turns of entangled coil segments at the points X and Y.

The different turns of entangled coil segments works to reduce the rotation-following capability to deteriorate the maneuverability so as to impede the good treatment against the diseased area if the guide wire has no fixedly-connected portion P between the flexible elongation core 2 and the helical spring tube 3.

Since it is possible to place the proximal region (L3) at a rear portion of the point X and at a front portion of the point Y on the leading front portion 1A, the guide wire 1 enables the manipulator to locate the proximal region (L3) at the proximal side of the point X, the distal side of the point Y and a middle portion between the points X and Y. This means that the flexible elongation core 2 and the helical spring tube 3 are integrally united concentrically to serve as a torque transmitter so as to solve the entangled coil segments appeared in relation to the points X and Y. This provides the guide wire 1 with a high torque transmissibility so as to overcome the damage on the vascular wall due to the reactionary force invited when the guide wire 1 is forcibly pushed.

Further, the above arragement enables the manipulator to a good steerability based on the high rotation-following capability represented upon manipulating the guide wire 1 within the somatic cavity.

The guide wire 1 was prepared to have two fixedly-connected portions P placed at regular intervals in the proximal region (L3) for an experimentation purpose. As evidenced in Fig. 14, the guide wire 1 was compared to the prior art guide wire in which the fixedly-connected portions P were not provided. When the rotational torque given to the two guide wires rotates at the proximal end side by 360 degrees, the transmitted torque rotates the distal end portion of the prior art guide wire by 53 degrees while rotating the distal end portion of the guide wire 1 by 257 degrees. This means the rotation-following capability of the guide wire 1 is approx. five times as good as that of the prior art guide wire.

The high rotation-following capability thus insured is based on the following mechanism.

When the helical spring tube 3 is subjected to the rotational torque, the helical spring tube 3 serves as a torsion spring in which the transmissible torque given to the helical spring tube 3 is in inverse proportion to the turns of the helical spring tube 3. Based on this theory, the turns of the helical spring tube 3 reduces to 1/2 times with the transmissible torque multiplied two times under the presence of one fixedly-connected portion P. The turns of the helical spring tube 3 reduces to 1/4 times with the transmissible torque multiplied four times under the presence of two fixedly-connected portions P.

Although it is sufficient to place at least one fixedly-connected portion P in the proximal region (L3) to achieve the high rotation-following capability, it is preferable to place the fixedly-connected portion P individually at the proximal side of the point X, the distal side of the point Y and the middle portion between the points X and Y. It is not preferable to provide the fixedly-connected portions P more than four pieces because it increases the bending rigidity of the proximal region (L3).

When applying the prior art guide wire to the right coronary artery (RCA) 15a, the guide wire makes its elevational portions in contact with an inner side and an outer side of the aortic arch 21 as designated by points X2 and Y2 in Fig. 13. This invites the same inconveniences as raised when the prior art guide wire is applied to the left main trunk (LMT) 15 in Fig. 15.

Upon inserting the catheter 18 into the aortic arch 21 through the brachiocephalic artery 22 as shown at brocken lines in Fig. 13, the guide wire bends and comes in contact with the inner side of the aortic arch 21 at the point Y2 so as to produce the above incovenience.

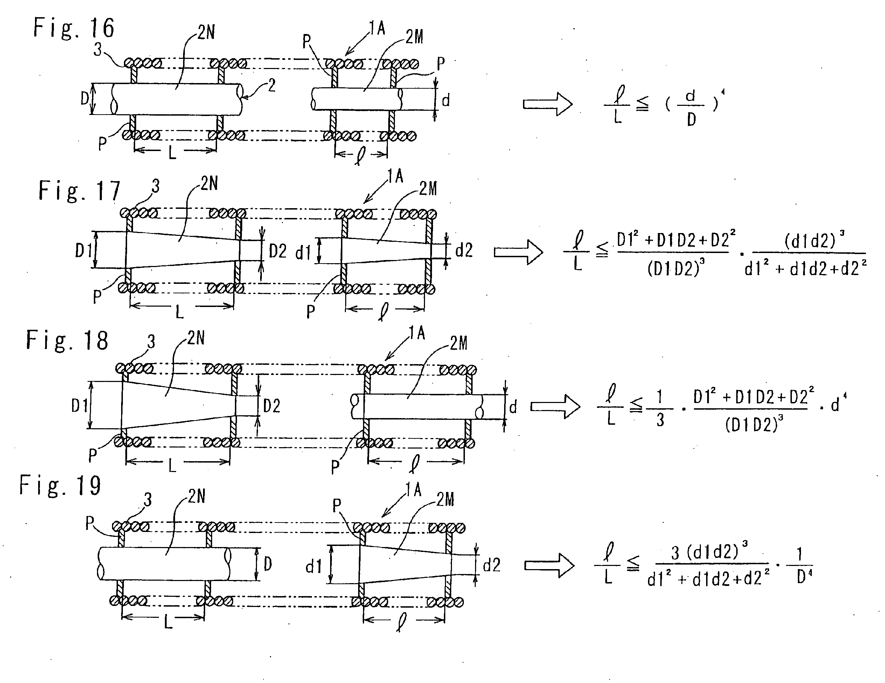

Figs. 16 through 19 show a sixth embodiment of the invention in which the helical spring tube 3 is placed around a diameter-increased portion 2N and a diameter-reduced portion 2M of the flexible elongation core 2.

As for a space opposed interval L ( ℓ ) of the opposed pair of the fixedly-connected portions P axially arranged along the flexible elongation core 2, the space opposed interval L ( ℓ ) is determined with a diametrical dimension as a reference level in which the opposed pair of the fixedly-connected portions P are located at the flexible elongation core 2. This forms a functionally equal structure which retains a uniform torque transmissibility and rotation-following capability, or forming a functionally gradient structure which gradually decreases the torque transmissibility and rotation-following capability from the proximal side to the distal side of the flexible elongation core 2.

The torsional rigidity of the space opposed interval L ( ℓ ) is in inverse proportion to the turns of the helical spring tube 3 between the pair of the fixedly-connected portions P, and at the same time, having a numerical relationship with a diametrical dimension of the flexible elongation core section between the opposed pair of the fixedly-connected portions P.

When the diameter-increased portion 2N (equi-diameter in the lengthwise direction) and the diameter-reduced portion 2M (equi-diameter in the lengthwise direction) have different diametrical dimensions D, d at the intervals L, ℓ, a ratio ( ℓ/L) is calculated based on the moment of inertia by raising a ratio (d/D) to 4th power. In order to achieve the above functionally equal or functionally gradient structure, the ratio ( ℓ /L) is determined to be equal to or less than a diametrical ratio (d/D) ⁴ as shown in Fig. 16.

When the diameter-increased portion 2N and the diameter-reduced portion 2M are of frustocone-shaped configuration, the ratio ( ℓ /L) is determined based on the strength of matertial as shown in Fig. 17.

In Fig. 17, denotations D1, D2 are major and minor diameters of the diameter-increased portion 2N and denotations d1, d2 are major and minor diameters of the diameter-reduced portion 2M.

When the diameter-increased portion 2N is of frustocone-shaped configuration and the diameter-reduced portion 2M are of equi-diametrical core, the ratio ( ℓ/L) is determined as shown in Fig. 18.

When the diameter-increased portion 2N is of equi-diametrical core and the diameter-reduced portion 2M are of frustocone-shaped configuration, the ratio ( ℓ/L) is determined as shown in Fig. 19.

If the flexible elongation core 2 has discontinuous portion, a diameter of which abruptly changes stepwisely within the space opposed interval L ( ℓ ), an average diameter is adopted when determining the ratio (ℓ /L). It is to be noted that the determination of the ratio (ℓ /L) is not necessarily applied to all the intervals (L, ℓ) along the leading front portion 1A of the guide wire 1.

Supplementarily mentioning about advantages commonly derived through the embodiments 1-6 of the invention, the manipulation curvedly deforms the leading front portion 1A to appear a clearances between the coil elements of the helical spring tube 3 at its outer elevational side upon inserting the guide wire 1 into the meanderous blood vessel. The helical spring tube 3 permits the blood streams to enter inside through the clearances, thus affecting on the fixedly-connected portions to generate a propelling force so as to move the guide wire 1 forward.

In this situation, the invasion of the leading front portion 1A into the blood vessel increases the speed of the blood streams, and each of the fixedly-connected portions P is affected by the blood streams. These factors amplifies the propelling force and making it possible to advance the guide wire 1 deeper into the blood vessel even if it is complicatedly and sinuously curved.

With the distal front portion 2A of the flexible elongation core 2 gradually decreasing diametrically as approaching the distal end T, an area increases progressively which the fixedly-connected portion P receives the propelling force as approaching the distal end T. This effectively assists the distal front portion 2A to advance into the blood vessel although the distal front portion 2A is likely to face a larger insertion resistance.

When the front end of the helical spring tube 3 is formed by the radiopaque material, the leading front portion 1A tends to hang down due to a difference of the specific gravity between the radiopaque front end and the other portion of the helical spring tube 3 (e.g., 21. 4 g/cm³ and 7.9 g/cm³ cited as specific gravities for platinum and stainless steel). With the leading front portion 1A free from the fixedly-connected portions P within 20 mm from the distal end T, it is possible to avoid the leading front portion 1A from inadvertently hanging down so as to favorably assist buoying it up in the blood streams.

Under the condition that the leading front portion gets stuck in the vascular stenosis portion 11 in the prior art guide wire as shown in Fig. 8, if the guide wire is forcibly rotated further, the rotational operation significantly twists the helical spring tube in front and in rear so as to require more rotational operation. The operation thus repeated would do an unfavorable deformation and a damage on the helical spring tube { 0.072 mm (diameter of the coil line element)} and the elongation core (0.031-0.049 mm in thickness).

As opposed to the prior art guide wire, due to the presence of the fixedly-connected portions P, it is sufficient to rotate the guide wire 1 stuck in the vascular stenosis portion 11 (Fig. 8) with the rotational torque given to the turns of the helical spring tube 3 merely corresponding to the interval S between the opposed pair of the fixedly-connected portions P.

This makes it easy to manipulatively rotate the guide wire 1 stuck in the vascular stenosis portion 11, and eliminates the unfavorable deformation and the damage on the leading front portion 1A, thus significantly ameliorating the treatment against the diseased area.

It is to be appreciated that instead of realizing the embodiments 1-6 individually, it is known for those versed in the art to appropriately combine the embodiments 1-6 upon putting into practice.

## Claims

1. A medical guide wire (1) including a flexible elongate core (2) having a distal front portion (2A), a proximal portion (2B) provided to be diametrically larger than said distal front portion (2A) and a leading front portion (1A) to which a helical spring tube (3) is inserted, both ends of which are secured to said flexible elongate core (2) ;
said distal front portion (2 A) of said flexible elongate core (2) is diametrically tapered or reduced progressively as approaching toward a distal end (T) of said flexible elongate core (2);
a non-integral region (LA) is provided to form an annual space between said flexible elongate core (2) and said helical spring tube (3) to extend by at least 20 mm axially from said distal end (T) of said flexible elongate core (2); **characterised in that**
an intermediate region (L2) is provided to form a group of fixedly-connected portions (P) between said flexible elongate core (2) and said helical spring tube (3) to axially extend by 50-125 mm from said distal end (T) of said flexible elongate core (2);
a proximal region (L3) is provided to form a group of fixedly-connected portions (P) between said flexible elongate core (2) and said helical spring tube (3) to axially extend by 125-300 mm from said distal end (T) of said flexible elongate core (3);
spans between said fixedly-connected portions (P) of said proximal region (L3) is greater than spans between said fixedly-connected portions (P) of said intermediate region (L2);
said fixedly-connected portions (P) are formed into a doughnut-shaped configuration to have 0.3-1.5 mm in breadth, and integrally connecting an inner surface of said helical spring tube (3) to an outer surface of said flexible elongate core (2);
wherein a space opposed interval of an opposed pair of said fixedly-connected portions (P) axially arranged along said flexible elongate core (2) is determined with a diametrical dimension as a reference level in which said opposed pair of said fixedly-connected portions (P) are located at said elongate core (2), and forming a structure which retains a uniform torque transmissibility and rotation-following capability, or forming a structure which gradually decreases the torque transmissibility and rotation-following capability from a proximal side to a distal side of said elongate core (2), so that one or combined ones selected from the group consisting of following dimensional relationship (i)-(iii).
(i) ℓ /L ≦ { ( D1² +D1 · D2+D2²) / ( D1 · D2)³ } · { (d1 · d2)³ / (d1 ² + d1 · d2+ d2²) } when said elongate core is a frustocone-shaped configuration at the space opposed interval L (ℓ) between the fixedly-connected portions (P),
where (D1) is a major diameter of a diameter-increased portion (2N) of said elongate core (2) at the space opposed interval (L), and (d1) is a major diameter of a diameter-reduced portion (2M) of said elongate core (2) at the space opposed interval ( ℓ ) between the fixedly-connected portions (P),
(D2) is a minor diameter of a diameter-increased portion (2N) of said elongate core (2) at the space opposed interval (L), and (d2) is a minor diameter of a diameter-reduced portion (2M) of said elongate core (2) at the space opposed interval (ℓ) between the fixedly-connected portions (P),
(ii) ℓ/L≦ (1/3) · ( (D1 ² + D1· D2+D2² )/(D1· D2) ³ } · d ⁴ when said elongate core (2) is a frustocone-shaped configuration at the space opposed interval (L), and equi-diametrical configuration at the space opposed interval ( ℓ ) between the fixedly-connected portions (P),
where (D1) is a major diameter of the diameter-increased portion (2N) of said elongate core (2) at the space opposed interval (L), and (D2) is a minor diameter of the diameter-increased portion (2N) of said elongate core (2) at the space opposed interval (L) between the fixedly-connected portions (P),
(d) is a diameter of the diameter-reduced portion (2M) of said elongate core (2) at the space opposed interval ( ℓ ) between the fixedly-connected portions (P),
(iii) ℓ/L≦ {3(d1 · d2)³ / (d1² + d1 · d2+d2²)} · ( 1/D⁴) when said elongate core (2) is a equi-diametrical configuration at the space opposed interval (L), and frustocone-shaped configuration at the space opposed interval ( ℓ ) between the fixedly-connected portions (P),
where (d1) is a major diameter of the diameter-reduced portion (2N) of said elongate core (2) at the space opposed interval ( ℓ ), and (d2) is a minor diameter of the diameter-reduced portion (2N) of said elongate core (2) at the space opposed interval ( ℓ ) between the fixedly-connected portions (P),
(D) is a diameter of the diameter-increased portion (2M) of said elongate core (2) at the space opposed interval (L) between the fixedly-connected portions (P),
in each case (i)-(iii), when said elongate core (2) has portions, diameters of which change within the space opposed interval (L, ℓ), an average diameter between these changed portions is adapted upon determining the ratio ( ℓ/L).

2. The medical guide wire (1) according to claim 1, wherein following dimensional relationship (iv) is added to be combined with one or more of said relationship (i)-(iii).
(iv) *ℓ* / L≦ (d/D)⁴ when said elongate core (2) is an equi-diametrical configuration at the space opposed interval (L, ℓ) between the fixedly-connected portions (P),
where (D) is a diameter of the diameter-increased portion (2N) of said elongate core (2) at the space opposed interval (L), and (d) is a diameter of the diameter-reduced portion (2M) of said elongate core (2) at the space opposed interval (ℓ) between the fixedly-connected portions (P).

3. The medical guide wire (1) according to claim 1 or 2, wherein said spans (S1, S2, S3,···, SN) between said fixedly-connected portions (P) of said intermediate region (L2) are arranged to be progressively reduced or increased dimensionally in a series fashion along an axial direction of said flexible elongate core (2).

4. The medical guide wire (1) according to claim 1 or 2, wherein said fixedly-connected portions (P) of said intermediate region (L2) are formed by a radiopaque material and arranged at regular intervals (S), and a front portion (3A) of said helical spring tube (3) and a rear portion (3B) of said helical spring tube (3) are made by different metals of a radiopaque material and a radiotransparent material, said different metals being connectedly bonded and wound to form a single helical structure, said front portion (3A) of said helical spring tube (3) being of the radiopaque material and having a helical length integral times greater than said span of said intermediate region (L2).

5. The medical guide wire (1) according to claim 1 or 2, wherein said fixedly-connected portions (P) of said intermediate region (L2) are formed by a radiopaque material to provide a plurality of unit portions (U) composed of smaller spans (SB) and larger spans (SA), and a front portion (3A) of said helical spring tube (3) and a rear portion (3B) of said helical spring tube (3) are made by different metals of a radiopaque material and a radiotransparent material so as to form a single helical structure, said front porion (3A) of said helical spring tube (3) being of the radiopaque material and having a helical length (L1) integral times greater than said smaller spans (SB).

6. The medical guide wire (1) according to claim 5, wherein spans of said fixedly-connected portions (P) of said intermediate region (L2) forms a plurality of said unit portions (U) composed of larger spans (SA) at proximal side of said flexible elongate core and smaller spans (SB) at distal side of said flexible elongate core (2).

7. The medical guide wire (1) according to any of claims 1-6, wherein number of said fixedly-connected portions (P) of said proximal region (L3) is in the range of 1-3.

## Patentansprüche

1. Medizinischer Führungsdraht (1) mit einem biegsamen länglichen Kern (2) mit einem distalen Vorderteil (2A), einem proximalen Teil (2B), der so vorgesehen ist, dass er diametral größer ist als der distale Vorderteil (2A), und einem führenden Vorderteil (1A), an den ein Schraubenfederrohr (3) angefügt ist, dessen beide Enden an dem biegsamen länglichen Kern (2) befestigt sind;
wobei der distale Vorderteil (2A) des biegsamen länglichen Kerns (2) bei der Annäherung in Richtung eines distalen Endes (T) des biegsamen länglichen Kerns (2) fortschreitend diametral verjüngt oder verkleinert ist;
wobei ein nicht-einteiliger Bereich (LA) vorgesehen ist, um einen ringförmigen Raum zwischen dem biegsamen länglichen Kern (2) und dem Schraubenfederrohr (3) so zu bilden, dass er sich um mindestens 20 mm axial vom distalen Ende (T) des biegsamen länglichen Kerns (2) erstreckt; **dadurch gekennzeichnet, dass**
ein Zwischenbereich (L2) vorgesehen ist, um eine Gruppe von fest verbundenen Teilen (P) zwischen dem biegsamen länglichen Kern (2) und dem Schraubenfederrohr (3) zu so bilden, dass sie sich um 50-125 mm vom distalen Ende (T) des biegsamen länglichen Kerns (2) axial erstrecken;
ein proximaler Bereich (L3) vorgesehen ist, um eine Gruppe von fest verbundenen Teilen (P) zwischen dem biegsamen länglichen Kern (2) und dem Schraubenfederrohr (3) so zu bilden, dass sie sich um 125-300 mm vom distalen Ende (T) des biegsamen länglichen Kerns (3) axial erstrecken;
Spannen zwischen den fest verbundenen Teilen (P) des proximalen Bereichs (L3) größer sind als Spannen zwischen den fest verbundenen Teilen (P) des Zwischenbereichs (L2);
die fest verbundenen Teile (P) zu einer ringförmigen Konfiguration gebildet sind, so dass sie eine Breite von 0,3-1,5 mm aufweisen, und eine innere Oberfläche des Schraubenfederrohrs (3) mit einer äußeren Oberfläche des biegsamen länglichen Kerns (2) einteilig verbinden;
wobei ein Raumgegenintervall eines gegenüberliegenden Paars der fest verbundenen Teile (P), die entlang des biegsamen länglichen Kerns (2) axial angeordnet sind, mit einer diametralen Abmessung als Bezugsniveau bestimmt ist, in dem das gegenüberliegende Paar der fest verbundenen Teile (P) am länglichen Kern (2) angeordnet sind, und eine Struktur gebildet wird, die eine gleichmäßige Drehmomentübertragungsfähigkeit und Drehfolgefähigkeit aufrechterhält, oder eine Struktur gebildet wird, die die Drehmomentübertragungsfähigkeit und Drehfolgefähigkeit von einer proximalen Seite zu einer distalen Seite des länglichen Kerns (2) allmählich verringert, so dass eine oder kombinierte, die aus der Gruppe ausgewählt ist (sind), die aus der folgenden Maßbeziehung (i)-(iii) besteht,
(i) ℓ/L ≤ {(D1² + D1 · D2 + D2²) / (D1 · D2)³} · {(d1 · d2)³ / (d1² + d1 · d2 + d2²)}, wenn der längliche Kern eine kegelstumpfförmige Konfiguration in dem Raumgegenintervall L (ℓ) zwischen den fest verbundenen Teilen (P) ist,
wobei (D1) ein größerer Durchmesser eines im Durchmesser vergrößerten Teils (2N) des länglichen Kerns (2) in dem Raumgegenintervall (L) ist und (d1) ein kleinerer Durchmesser eines im Durchmesser verringerten Teils (2M) des länglichen Kerns (2) in dem Raumgegenintervall (ℓ) zwischen den fest verbundenen Teilen (P) ist,
(D2) ein kleinerer Durchmesser eines im Durchmesser vergrößerten Teils (2N) des länglichen Kerns (2) in dem Raumgegenintervall (L) ist, und (d2) ein kleinerer Durchmesser eines im Durchmesser verringerten Teils (2M) des länglichen Kerns (2) in dem Raumgegenintervall (ℓ) zwischen den fest verbundenen Teilen (P) ist,
(ii) ℓ/L ≤ (1/3) · {(D1² + D1 · D2 + D2²) / (D1 · D2)³} · d⁴,
wobei der längliche Kern (2) eine kegelstumpfförmige Konfiguration in dem Raumgegenintervall (L) und eine diametral gleiche Konfiguration in dem Raumgegenintervall (ℓ) zwischen den fest verbundenen Teilen (P) ist,
wobei (D1) ein größerer Durchmesser des im Durchmesser vergrößerten Teils (2N) des länglichen Kerns (2) in dem Raumgegenintervall (L) ist, und (D2) ein kleinerer Durchmesser des im Durchmesser vergrößerten Teils (2N) des länglichen Kerns (2) in dem Raumgegenintervall (L) zwischen den fest verbundenen Teilen (P) ist,
(d) ein Durchmesser des im Durchmesser verringerten Teils (2M) des länglichen Kerns (2) in dem Raumgegenintervall (ℓ) zwischen den fest verbundenen Teilen (P) ist,
(iii) ℓ/L ≤ {3 (d1 · d2)³ /(d1² + d1 · d2 + d2 ²)} · (1/D⁴) , wenn der längliche Kern (2) eine diametral gleiche Konfiguration in dem Raumgegenintervall (L) und eine kegelstumpfförmige Konfiguration in dem Raumgegenintervall (ℓ) zwischen den fest verbundenen Teilen (P) ist,
wobei (d1) ein größerer Durchmesser des im Durchmesser verringerten Teils (2N) des länglichen Kerns (2) in dem Raumgegenintervall (ℓ) ist und (d2) ein kleinerer Durchmesser des im Durchmesser verringerten Teils (2N) des länglichen Kerns (2) in dem Raumgegenintervall (ℓ) zwischen den fest verbundenen Teilen (P) ist,
(D) ein Durchmesser des im Durchmesser vergrößerten Teils (2M) des länglichen Kerns (2) in dem Raumgegenintervall (L) zwischen den fest verbundenen Teilen (P) ist,
in jedem Fall (i)-(iii), wenn der längliche Kern (2) Teile aufweist, deren Durchmesser sich innerhalb des Raumgegenintervalls (L, ℓ) ändern, ein mittlerer Durchmesser zwischen diesen geänderten Teilen bei der Bestimmung des Verhältnisses (ℓ/L) angepasst wird.

2. Medizinsicher Führungsdraht (1) nach Anspruch 1, wobei die folgende Maßbeziehung (iv) hinzugefügt wird, damit sie mit einer oder mehreren der Beziehungen (i)-(iii) kombiniert wird,
(iv) ℓ/L ≤ (d/D)⁴, wenn der längliche Kern (2) eine diametral gleiche Konfiguration in dem Raumgegenintervall (L, ℓ) zwischen den fest verbundenen Teilen (P) ist,
wobei (D) ein Durchmesser des im Durchmesser vergrößerten Teils (2N) des länglichen Kerns (2) in dem Raumgegenintervall (L) ist und (d) ein Durchmesser des im Durchmesser verringerten Teils (2M) des länglichen Kerns (2) in dem Raumgegenintervall (ℓ) zwischen den fest verbundenen Teilen (P) ist.

3. Medizinischer Führungsdraht (1) nach Anspruch 1 oder 2,
wobei die Spannen (S1, S2, S3, ..., SN) zwischen den fest verbundenen Teilen (P) des Zwischenbereichs (L2) so angeordnet sind, dass sie in einer Reihenweise entlang einer axialen Richtung des biegsamen länglichen Kerns (2) maßlich fortschreitend verkleinert oder vergrößert sind.

4. Medizinischer Führungsdraht (1) nach Anspruch 1 oder 2,
wobei die fest verbundenen Teile (P) des Zwischenbereichs (L2) aus einem strahlungsundurchlässigen Material gebildet sind und in regelmäßigen Intervallen (S) angeordnet sind, und ein Vorderteil (3A) des Schraubenfederrohrs (3) und ein Hinterteil (3B) des Schraubenfederrohrs (3) aus verschiedenen Metallen eines strahlungsundurchlässigen Materials und eines strahlungsdurchlässigen Materials hergestellt sind, wobei die verschiedenen Metalle verbunden geklebt und gewunden sind, um eine einzige schraubenförmige Struktur zu bilden, wobei der Vorderteil (3A) des Schraubenfederrohrs (3) aus dem strahlungsundurchlässigen Material besteht und eine Schraubenlänge aufweist, die ganzzahlige Male größer ist als die Spanne des Zwischenbereichs (L2).

5. Medizinischer Führungsdraht (1) nach Anspruch 1 oder 2,
wobei die fest verbundenen Teile (P) des Zwischenbereichs (L2) aus einem strahlungsundurchlässigen Material gebildet sind, um eine Vielzahl von Einheitsteilen (U) bereitzustellen, die aus kleineren Spannen (SB) und größeren Spannen (SA) bestehen, und ein Vorderteil (3A) des Schraubenfederrohrs (3) und ein Hinterteil (3B) des Schraubenfederrohrs (3) aus verschiedenen Metallen eines strahlungsundurchlässigen Materials und eines strahlungsdurchlässigen Materials hergestellt sind, um eine einzelne schraubenförmige Struktur zu bilden, wobei der Vorderteil (3A) des Schraubenfederrohrs (3) aus dem strahlungsundurchlässigen Material besteht und eine Schraubenlänge (L1) aufweist, die ganzzahlige Male größer ist als die kleineren Spannen (SB).

6. Medizinischer Führungsdraht (1) nach Anspruch 5, wobei die Spannen der fest verbundenen Teile (P) des Zwischenbereichs (L2) eine Vielzahl der Einheitsteile (U) bilden, die aus größeren Spannen (SA) auf der proximalen Seite des biegsamen länglichen Kerns und kleineren Spannen (SB) auf der distalen Seite des biegsamen länglichen Kerns (2) bestehen.

7. Medizinischer Führungsdraht (1) nach einem der Ansprüche 1-6, wobei die Anzahl der fest verbundenen Teile (P) des proximalen Bereichs (L3) im Bereich von 1-3 liegt.

## Revendications

1. Fil guide médical (1) comprenant une âme allongée flexible (2) ayant une partie avant distale (2A), une partie proximale (2B) prévue pour être diamétralement supérieure à ladite partie avant distale (2A) et une partie avant d'attaque (1A) dans laquelle un tube de ressort hélicoïdal (3) est inséré, dont les deux extrémités sont fixées sur ladite âme allongée flexible (2) ;
ladite partie distale (2A) de ladite âme allongée flexible (2) est diamétralement progressivement rétrécie ou progressivement réduite lorsqu'elle s'approche de l'extrémité distale (T) de ladite âme allongée flexible (2) ;
une région non solidaire (LA) est prévue pour former un espace annulaire entre ladite âme allongée flexible (2) et ledit tube du ressort hélicoïdal (3) pour s'étendre sur au moins 20 mm de manière axiale à partir de ladite extrémité distale (T) de ladite âme allongée flexible (2) ; **caractérisé en ce que** :
une région intermédiaire (L2) est prévue pour former un groupe de parties raccordées de manière fixe (P) entre ladite âme allongée flexible (2) et ledit tube de ressort hélicoïdal (3) pour s'étendre de manière axiale sur 20-25 mm à partir de ladite extrémité distale (T) de ladite âme allongée flexible (2) ;
une région proximale (L3) est prévue pour former un groupe de parties raccordées de manière fixe (P) entre ladite âme allongée flexible (2) et ledit tube de ressort hélicoïdal (3) pour s'étendre de manière axiale sur 125-300 mm de ladite extrémité distale (T) de ladite âme allongée flexible (3),
des portées entre lesdites parties raccordées de manière fixe (P) de ladite région proximale (L3) sont supérieures aux portées entre lesdites parties raccordées de manière fixe (P) de ladite région intermédiaire (L2) ;
lesdites parties raccordées de manière fixe (P) sont formées selon une configuration en forme de beignet pour avoir une larguer de 0,3-1,5 mm, et raccordant de manière solidaire une surface interne dudit tube de ressort hélicoïdal (3) à une surface externe de ladite âme allongée flexible (2) ;
dans lequel un intervalle opposé espacé d'une paire opposée desdites parties raccordées de manière fixe (P) agencée de manière axiale le long de ladite âme allongée flexible (2) est déterminé avec la dimension diamétrale comme un niveau de référence dans lequel ladite paire opposée desdites parties raccordées de manière fixe (P) est située au niveau de ladite âme allongée (2), et formant une structure qui conserve une transmission de couple uniforme et une capacité de poursuite de rotation, ou formant une structure qui diminue progressivement la transmission de couple et la capacité de poursuite de rotation d'un côté proximal à un côté distal de ladite âme allongée (2), de sorte qu'un élément ou des éléments combinés choisis dans le groupe se composant des relations dimensionnelles suivantes (i) - (iii) :
(i) l/L ≤ { D1² + D1 · D2 + D2²) / (D1 · D2)³ } · {(d1 · d2) ³ /(d1² + d1 · d2 + d2²) } lorsque ladite âme allongée a une configuration tronconique au niveau de l'intervalle opposé espacé L(1) entre les parties raccordées de manière fixe (P),
où (D1) est un diamètre majeur d'une partie de diamètre augmentée (2N) de ladite âme allongée (2) au niveau de l'intervalle opposé espacé (L), et (d1) est un diamètre majeur d'une partie réduite en diamètre (2M) de ladite âme allongée (2) au niveau de l'intervalle opposé espacé (1) entre les parties raccordées de manière fixe (P),
(D2) est un diamètre mineur d'une partie augmentée en diamètre (2N) de ladite âme allongée (2) au niveau de l'intervalle opposé espacé (L), et (d2) est un diamètre mineur d'une partie réduite en diamètre (2M) de ladite âme allongée (2) au niveau de l'intervalle opposé espacé (1) entre les parties raccordées de manière fixe (P),
(ii) l/L ≤ (1/3) · { D1² + D1 · D2 + D2²) / (D1 · D2)³} · d⁴ lorsque ladite âme allongée (2) a une configuration de forme tronconique au niveau de l'intervalle opposé espacé (L), et une configuration équidiamétrale au niveau de l'intervalle opposé espacé (1) entre les parties raccordées de manière fixe (P),
où (D1) est un diamètre majeur de la partie augmentée en diamètre (2N) de ladite âme allongée (2) au niveau de l'intervalle opposé espacé (N), et (D2) est un diamètre mineur de la partie augmentée en diamètre (2N) de ladite âme allongée (2) au niveau de l'intervalle opposé espacé (L) entre les parties raccordées de manière fixe (P),
(d) est un diamètre de la partie réduite en diamètre (2M) de ladite âme allongée (2) au niveau de l'intervalle opposé espacé (1) entre les parties raccordées de manière fixe (P),
(iii) l/L ≤ (3(d1 · d2)³/ (d1² + d1 · d2 + d2²)} · (1/D⁴) lorsque ladite âme allongée (2) est une configuration équidiamétrale au niveau de l'intervalle opposé espacé (L), et une configuration de forme tronconique au niveau de l'intervalle opposé espacé (1) entre les parties raccordées de manière fixe (P),
où (d1) est un diamètre majeur de la partie réduite en diamètre (2N) de ladite âme allongée (2) au niveau de l'intervalle opposé espacé (1) et (d2) est un diamètre mineur de la partie réduite en diamètre (2N) de ladite âme allongée (2) au niveau de l'intervalle opposé espacé (1) entre les parties raccordées de manière fixe (P),
(D) est un diamètre de la partie augmentée en diamètre (2M) de ladite âme allongée (2) au niveau de l'intervalle opposé espacé (L) entre les parties raccordées de manière fixe (P),
dans chaque cas (i)-(iii), lorsque ladite âme allongée (2) a des parties, dont les diamètres changent dans l'intervalle opposé espacé (L, 1), un diamètre moyen entre ses parties modifiées est adapté suite à la détermination du rapport (1/L) .

2. Fil guide médical (1) selon la revendication 1, dans lequel la relation dimensionnelle (iv) suivante est ajoutée pour être combinée à une ou plusieurs desdites relations (i)-(iii).
(iv) l/L ≤ (d/D)⁴ lorsque ladite âme allongée (2) a une configuration équidiamétrale au niveau de l'intervalle opposé espacé (L,1) entre les parties raccordées de manière fixe (P),
où (D) est un diamètre de la partie augmentée en diamètre (2N) de ladite âme allongée (2) au niveau de l'intervalle opposé espacé (L), et (d) est un diamètre de la partie réduite en diamètre (2M) de ladite âme allongée (2) au niveau de l'intervalle opposé espacé (1) entre les parties raccordées de manière fixe (P).

3. Fil guide médical (1) selon la revendication 1 ou 2,
dans lequel lesdites portées (S1, S2, S3,..., SN) entre lesdites parties raccordées de manière fixe (P) de ladite région intermédiaire (L2) sont agencées pour être progressivement réduites ou augmentées de manière dimensionnelle en série le long d'une direction axiale de ladite âme allongée flexible (2).

4. Fil guide médical (1) selon la revendication 1 ou 2,
dans lequel lesdites parties raccordées de manière fixe (P) de ladite région intermédiaire (L2) sont formées avec un matériau radio-opaque et agencées à des intervalles réguliers (S), et une partie avant (3A) dudit tube de ressort hélicoïdal (3) et une partie arrière (3B) dudit tube de ressort hélicoïdal (3) sont réalisées avec des métaux différents d'un matériau radio-opaque et d'un matériau radio-transparent, lesdits métaux différents étant collés par raccordement et enroulés pour former une structure hélicoïdale unique, ladite partie avant (3A) dudit tube de ressort hélicoïdal (3) étant réalisée avec un matériau radio-opaque et ayant une longueur hélicoïdale plusieurs fois supérieure à ladite portée de ladite région intermédiaire (L2).

5. Fil guide médical (1) selon la revendication 1 ou 2,
dans lequel lesdites parties raccordées de manière fixe (P) de ladite région intermédiaire (L2) sont formées par un matériau radio-opaque pour proposer une pluralité de parties unitaires (U) composées de plus petites portées (SB), et de plus grandes portées (SA) et une partie avant (3A) dudit tube de ressort hélicoïdal (3) et une partie arrière (3B) dudit tube de ressort hélicoïdal (3) sont réalisées avec des métaux différents d'un matériau radio-opaque et d'un matériau radio-transparent afin de former une structure hélicoïdale unique, ladite partie avant (3A) dudit tube de ressort hélicoïdal (3) étant réalisée avec un matériau radio-opaque et ayant une longueur hélicoïdale (L1) plusieurs fois supérieure auxdites plus petites portées (SB).

6. Fil guide médical (1) selon la revendication 5, dans lequel les portées desdites parties raccordées de manière fixe (P) de ladite région intermédiaire (L2) forment une pluralité desdites parties unitaires (U) composées de plus grandes portées (SA) au niveau du côté proximal de ladite âme allongée flexible et des plus petites portées (SB) au niveau du côté distal de ladite âme allongée flexible (2).

7. Fil guide médical (1) selon l'une quelconque des revendications 1 à 6, dans lequel le nombre desdites parties raccordées de manière fixe (P) de ladite région proximale (L3) est dans la plage de 1-3.
